# EUROPÄISCHE PATENTSCHRIFT

(11) **EP 1 843 801 B1**
(45) Veröffentlichungstag und Bekanntmachung des Hinweises auf die Patenterteilung: **18.07.2012**
(21) Anmeldenummer: 06722981.5
(22) Anmeldetag: 18.01.2006
(51) Int. Cl.: A61L 27/04, A61L 27/42

(54) **ABSORBIERBARES MEDIZINISCHES IMPLANTAT AUS FASERVERSTÄRKTEM MAGNESIUM ODER FASERVERSTÄRKTEN MAGNESIUMLEGIERUNGEN**
ABSORBABLE MEDICAL IMPLANT MADE OF FIBER-REINFORCED MAGNESIUM OR FIBER-REINFORCED MAGNESIUM ALLOYS
IMPLANT MEDICAL RESORBABLE EN MAGNESIUM RENFORCE PAR DES FIBRES OU EN ALLIAGES DE MAGNESIUM RENFORCES PAR DES FIBRES

(30) Priorität: 20.01.2005 DE 102005003188
(43) Veröffentlichungstag der Anmeldung: 17.10.2007
(73) Patentinhaber: Biotronik VI Patent AG, 6340 Baar (CH)
(72) Erfinder: KAPPELT, Gerhard, 91080 Uttenreuth (DE); LÖFFLER, Jörg, CH-8049 Zürich (CH); MÜLLER, Heinz, 91052 Erlangen (DE); UGGOWITZER, Peter, CH-8913 Ottenbach (CH)
(74) Vertreter: Lindner-Vogt, Karin L.
(86) Internationale Anmeldenummer: PCT/EP2006/000565
(87) Internationale Veröffentlichungsnummer: WO 2006/077154

(56) Entgegenhaltungen:
- EP-A- 1 216 717
- DE-A1- 10 118 603
- DE-A1- 19 731 021
- US-A- 4 279 249

## Beschreibung

Die Erfindung ist ein medizinisches Implantat, bestehend aus einem Verbundwerkstoff, bestehend aus einer kristallinen Matrix aus biokorrodierbarem Magnesium oder Magnesiumlegierungen, welche mit amorphen oder nanokristallinen Fasern aus einer magnesiumhaltigen, biokorrodierbaren Legierung, einer anderen biokorrodierbaren Legierung, oder einem biologisch nicht abbaubaren Faserwerkstoff verstärkt ist.

Medizinische Implantate unterschiedlichster Verwendungen sind in großer Vielfalt aus dem Stand der Technik bekannt. Ein gemeinsames Ziel bei der Realisation moderner medizinischer Implantate ist eine hohe Biokompatibilität, dass heißt ein hoher Grad an Gewebeverträglichkeit des in den Körper eingesetzten Medizinproduktes. Häufig ist nur eine temporäre Anwesenheit des Implantats im Körper zur Erfüllung des medizinischen Zweckes erforderlich. Implantate aus Werkstoffen, die sich nicht im Körper abbauen, sind wieder zu entfernen, da es mittel- und langfristig auch bei hoch biokompatiblen permanenten Werkstoffen zu Abstoßungsreaktionen des Körpers kommen kann.

Ein Ansatz zur Lösung vorgenannter Problematik besteht darin, das Implantat ganz oder in Teilen aus einem biokorrodierbaren Werkstoff zu formen. Unter Biokorrosion werden vorliegend mikrobielle Vorgänge oder schlicht durch die Anwesenheit von Körpermedien bedingte Prozesse verstanden, die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper resorbiert, wobei geringe Rückstände tolerierbar sind.

Biokorrodierbare Werkstoffe wurden unter anderem auf Basis von Polymeren synthetischer Natur oder natürlichem Ursprungs entwickelt. Aufgrund der Materialeigenschaften, aber auch insbesondere der Abbauprodukte der synthetischen Polymere ist der Einsatz biodegradierbarer Polymere noch deutlich limitiert. So müssen beispielsweise orthopädische Implantate häufig hohen mechanischen Beanspruchungen standhalten und vaskuläre Implantate, z.B. Stents, je nach Design sehr speziellen Anforderungen an E-Modul, Brüchigkeit und Formbarkeit genügen.

Ein viel versprechender Lösungsansatz sieht dazu den Einsatz biokorrodierbarer Metalllegierungen vor. So wird in der DE 197 31 021 A1 vorgeschlagen, medizinische Implantate aus einem metallischen Werkstoff zu formen, dessen Hauptbestandteil aus der Gruppe Alkalimetalle, Erdalkalimetalle, Eisen, Zink und Aluminium zu wählen ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90 %, Yttrium 3,7 - 5,5 %, Seltenerdmetallen 1,5 - 4,4 % und Rest < 1 % bekannt, die sich insbesondere zur Herstellung einer Endoprothese, z. B. in Form eines selbstexpandierenden oder ballonexpandierbaren Stent, eignet. Ungeachtet der erreichten Fortschritte auf dem Gebiet biokorrodierbarer Metalllegierungen sind auch die bisher bekannten Legierungen aufgrund ihrer Materialeigenschaften, wie Festigkeit und Korrosionsverhalten, nur beschränkt einsatzfähig. Insbesondere die relativ rasche Biokorrosion von Magnesiumlegierungen und deren im Vergleich zu anderen metallischen Werkstoffen niedrigere Festigkeit bedingt eine Limitierung des Einsatzgebietes. Als innovativer Ansatz wird in der DE 10 2005 003 188 A1 ein medizinisches Implantat beschrieben, das aus einer biokorrodierbaren amorphen oder nanokristallinen Legierung besteht oder diese enthält. Hiermit ist zwar nun die Konstruktion von Implantaten aus Magnesium und Magnesiumlegierungen möglich, welche eine im Vergleich zu kristallinem Magnesium und Magnesiumlegierungen erhöhte Festigkeit besitzen. Leider erkauft man sich jedoch diesen Vorteil in der Regel durch einen Verlust an Verformbarkeit und Wechsellastfestigkeit aufgrund erhöhter Biegesteifigkeit. Dies schränkt wiederum den Einsatzbereich dieser Legierungen stark ein.

Der vorliegenden Erfindung liegt die Aufgabe zugrunde, einen Werkstoff bereitzustellen, der eine insbesondere mit den bekannten biokorrodierbaren Magnesiumlegierungen vergleichbare hohe Biokompatibilität aufweist, aber verbesserte Materialeigenschaften für den Einsatz in Implantaten mit sich bringt. Wenn möglich, soll das Einsatzgebiet biokorrodierbarer Metalllegierungen in Implantaten erweitert werden.

Die Aufgabe wird durch das medizinische Implantat mit den in Anspruch 1 genannten Merkmalen gelöst.

Der Erfindung liegt die Erkenntnis zugrunde, medizinische Implantate aus kristallinem Magnesium oder Magnesiumlegierungen gezielt je nach mechanischer Beanspruchung ganz oder in Teilbereichen mit Verstärkungsfasern aus einer biokorrodierbaren, amorphen oder nanokristallinen Legierung mit einer Hauptkomponente ausgewählt aus der Gruppe Mg, Ca, Fe oder Y herzustellen. Die amorphen oder nanokristallinen Legierungen zeichnen sich gegenüber den bekannten kristallinen Metalllegierungen durch ihre in der Regel erhöhte Festigkeit und ein verzögertes in vivo Korrosionsverhalten aus. Durch das Einbetten von amorphen oder nanokristallinen Fasern in eine kristalline und damit leichter verformbare Matrix entsteht ein Verbundwerkstoff mit völlig neuen mechanischen Eigenschaften. Die amorphen oder nanokristallinen Fasern werden von der kristallinen Matrix vollflächig gestützt. Für das Implantat selbst resultiert hieraus eine erheblich höhere Elastizität im Vergleich zu einem Implantat, das ausschließlich aus amorphen oder nanokristallinen Legierungen gefertigt wird oder größere zusammenhängende Volumenanteile an diesen Legierungen enthält. Gleichzeitig bleibt der durch die amorphe und nanokristalline Struktur der Fasern verursachte Zugewinn an Festigkeit größtenteils erhalten, bzw. wird sogar vom dadurch entstehenden Verbundwerkstoff durch die zusätzliche Stützwirkung auf die Fasern noch übertroffen. Die Fasern können zusätzlich in ihrem Verlauf optimal den mechanischen Beanspruchungen des Bauteils angepasst werden.

Unter Hauptkomponente einer Legierung wird erfindungsgemäß die Legierungskomponente verstanden, deren Gewichtsanteil an der Legierung am höchsten ist. In den nachfolgend noch näher beschriebenen bevorzugten Legierungszusammensetzungen sind alle weiteren Anteile der Legierungskomponenten immer so vorgegeben, dass jeweils Mg, Ca, Fe oder Y den höchsten Gewichtsanteil hat.

Amorphe Legierungen im erfindungsgemäßen Sinne zeichnen sich dadurch aus, dass sie keine Kristallstruktur ausbilden und das Material in einer Art Anordnung ohne Periodizität, ohne Fernordnung, ähnlich den Atomen in einer Schmelze, allerdings mit fixierten atomaren Positionen verbleibt. Amorphe Legierungen werden auch als metallische Gläser bezeichnet (Engl. metallic glasses, glassy metals, amorphous metals). Auch wenn die Legierungen als amorph bezeichnet werden, so besitzen sie doch immer eine ausgeprägte Nahordnung sowohl topologisch, wie auch chemisch, die häufig der der entsprechenden kristallinen Gleichgewichtsphase ähnelt.

Nanokristalline Legierungen sind nicht völlig amorph, sondern enthalten einzelne Kristalle, deren Größe vorliegend als kleiner oder gleich 100 nm definiert wird. Auch nanokristalline Legierungen zeichnen sich durch von der kristallinen Gleichgewichtsphase abweichende chemische und topologische Eigenschaften aus.

Durch verschiedene, aus dem Stand der Technik bekannte Herstellungsverfahren kann verhindert werden, dass sich eine Kristallstruktur ausbildet und das Material im amorphen oder nanokristallinen Zustand verbleibt. Massiv geformte Halb- und Fertigwaren aus amorphen oder nanokristallinen Legierungen (auch Massivgläser genannt; Engl. bulk metallic glasses (BMG)) können beispielsweise durch schnelles Abschrecken einer Schmelze oder eines Dampfes hergestellt werden. Beim sogenannten Schmelzspinnen wird das flüssige Metall auf einen rasch rotierenden, gekühlten Zylinder geführt. Das sog. Schwebschmelzen (engl. levitation melting) befindet sich noch in der Entwicklungsphase. Hierbei wird ein Vorformling aus den einzelnen Legierungselementen im schwebenden Zustand im Vakuum erschmolzen. Aus diesem schwebenden Schmelzetropfen kann mittels kontinuierlicher Entnahme bei gleichmäßiger Abzugsgeschwindigkeit eine Faser gezogen werden.

Materialeigenschaften und Herstellungsverfahren für magnesiumhaltige amorphe Legierungen beschreiben (i) A. Inoue und T. Masumoto, Material Science and Engineering, A173 (1993) pp. 1 to 8; (ii) G. Yuan, T. Zhang und A. Inoue, Materials Transactions, Vol. 44, No. 11 (2003) pp. 2271 to 2275 und (iii) W.M. Rainforth und H. Jones, Scripta Materialia, Vol. 37, No. 3 (1997) pp. 311 to 314.

Theoretisch kann jede Legierung durch geeignete Verfahrensführung in amorpher oder nanokristalliner Modifikation erhalten werden. In der Praxis und für die Zwecke der Erfindung besonders geeignete Legierungen zeichnen sich durch ein günstiges Verhältnis von Glasübergangstemperatur T_{g} und Temperatur der Schmelze Tₗ aus (als Resultat des bekannten Turnbull-Kriteriums: D. Turnbull, Metall Trans B (1981) 271). Es ist weiterhin bekannt, dass der Zusatz von Legierungskomponenten zur Ausbildung eutektischer Phasen in den Schmelzen führen kann und dass verstärkte chemische und topologische Unterschiede zwischen den einzelnen Legierungsbestandteilen, z. B. in Atomgröße und im Bindungsverhalten, eine Kristallbildung bei der Abkühlung der Schmelze verhindern können.

Zur Auffindung geeigneter Legierungssysteme kann auf Datensammlungen von Phasendiagrammen binärer oder ternärer Legierungen zurückgegriffen werden (Massalski T.B., Okamoto H., Subramanian P.R., Kacprizak L., Binary alloy phase diagrams, vols. 1 to 3, Materials Park (OH): ASM International, 1990/1; Villars P., Prince A., Okamoto H., Handbook of ternary alloy phase diagrams, vols. 1 to 10, Materials Park (OH): ASM International, 1995). Ein weiterer Ansatz sieht vor, Schmelzen mit den in Frage kommenden Legierungskomponenten in Gegenwart hoher gravimetrischer Kräfte langsam abzukühlen. Dies kann dadurch erreicht werden, dass die Schmelzen in Tiegeln bereitgestellt werden, die von einer Zentrifuge aufgenommen werden und während des Zentrifugierens die Temperatur langsam abgesenkt wird (Löffler J. F., Johnson W. L., Intermetallics 10 (2002), pp. 1167 to 1175; Löffler J. F., Intermetallics 11 (2003), pp. 529 to 540; Löffler J. F., Peker A., Bossuyt S., Johnson W. L., Materials Science and Engineering A 375 - 377 (2004) pp. 341 to 345). Demnach ist es dem Fachmann mit Rückgriff auf die Literaturwerte oder die genannten gravimetrischen Untersuchungsmethoden ohne weiteres möglich, Eutektika oder dem Eutektika nahe liegende Zusammensetzungen zu identifizieren, die besonders stabile amorphe oder nanokristalline Legierungen bilden können.

Die amorphen oder nanokristallinen Legierungen sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht.

Vorzugsweise sind vaskuläre Implantate in Form von Stents derart hinsichtlich der eingesetzten biokorrodierbaren amorphen oder nanokristallinen Legierungen für die Verstärkungsfasern auszulegen, dass eine mechanische Integrität des Implantats für 2 - 20 Wochen aufrechterhalten wird. Implantate als Okkluder sind so hinsichtlich der biokorrodierbaren amorphen oder nanokristallinen Legierungen für die Verstärkungsfasern auszulegen, dass die mechanische Integrität des Implantats für 6 - 12 Monate aufrechterhalten wird. Orthopädische Implantate für die Osteosynthese sind hinsichtlich der biokorrodierbaren amorphen Legierungszusammensetzung für die Verstärkungsfasern so auszulegen, dass die mechanische Integrität des Implantats für 6 - 36 Monate aufrechterhalten wird.

Besonders bevorzugt sind:
(a) Eine Legierung der Zusammensetzung der Formel MgZnX, bei der (i) ein Anteil von Zn an der Legierung 10 - 40 Gew.% beträgt und (ii) X für ein oder mehrere Elemente ausgewählt aus der Gruppe Ln, Y, Si, Al, Ca steht und ein Anteil von X an der Legierung 0 - 20 Gew.% beträgt.
(b) Eine Legierung der Zusammensetzung MgZn, mit einem Anteil von Zn an der Legierung von 26 - 32 Gew.%, insbesondere 28,1 Gew.%.
(c) Eine Legierung der Zusammensetzung MgZnLn, bei der ein Anteil von Zn an der Legierung 10 - 40 Gew.% und ein Anteil von Ln an der Legierung 1 - 12 Gew.% beträgt.
(d) Eine Legierung der Zusammensetzung MgZnY, bei der ein Anteil von Zn an der Legierung 10 - 40 Gew.% und ein Anteil von Y an der Legierung 1 - 12 Gew.% beträgt.
(e) Eine Legierung der Zusammensetzung MgZnY, bei der ein Anteil von Zn an der Legierung 13 - 17 Gew.% und ein Anteil von Y an der Legierung 8 - 12 Gew.% beträgt.
(f) Eine Legierung der Zusammensetzung MgZnY, bei der ein Anteil von Zn an der Legierung 15 Gew.% und ein Anteil von Y an der Legierung 10 Gew.% beträgt.
(g) Eine Legierung der Zusammensetzung MgZnSi, bei der ein Anteil von Si an der Legierung 0,01 - 2 Gew.% und ein Anteil von Zn an der Legierung 10 - 40 Gew.% beträgt.
(h) Eine Legierung der Zusammensetzung MgZnAl, bei der ein Anteil von Al an der Legierung 0,01 - 20 Gew.% beträgt und ein Anteil von Zn an der Legierung 10 - 40 Gew.% beträgt.
(i) Eine Legierung der Zusammensetzung MgZnCa, bei der ein Anteil von Ca an der Legierung 0,01 - 20 Gew.% und ein Anteil von Zn an der Legierung 10 - 40 Gew.% beträgt.
(j) Eine Legierung der Zusammensetzung MgY, bei der ein Anteil von Y an der Legierung 1 - 30 Gew.% beträgt.
(k) Eine Legierung der Zusammensetzung MgY, bei der ein Anteil von Y an der Legierung 7 - 11 Gew.% beträgt.
(l) Eine Legierung der Zusammensetzung MgY, bei der ein Anteil von Y an der Legierung 8,9 Gew.% beträgt.
(m) Eine Legierung der Zusammensetzung MgYLn, bei der ein Anteil von Y an der Legierung 1 - 30 Gew.% und ein Anteil von Ln an der Legierung 1 - 12 Gew.% beträgt.
(n) Eine Legierung der Zusammensetzung MgCaAl, bei der ein Anteil von Ca an der Legierung 0,01 - 20 Gew.% und ein Anteil von Al an der Legierung 0,01 - 20 Gew.% beträgt.
(o) Eine Legierung der Zusammensetzung MgCaSi, bei der ein Anteil von Ca an der Legierung 0,01 - 20 Gew.% und ein Anteil von Si an der Legierung 0,01 - 2 Gew.% beträgt.

Die Legierungen (a) - (o) scheinen ersten experimentellen Versuchen nach besonders geeignet, den Erfordernissen an die Materialbeschaffenheit medizinischer Implantate, insbesondere der Biokompatibilität zu genügen.

Unter der Sammelbezeichnung "Lanthanoide" werden vorliegend die 14 auf Lanthan folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Es wird das Symbol Ln für die Lanthanoide verwendet.

Die erfindungsgemäßen medizinischen Implantate enthalten vorzugsweise Fasern oder Drähte aus der Legierung, da sich diese fertigungstechnisch einfach herstellen und verarbeiten lassen und die Gefahr des Auskristallisierens gering gehalten wird. Die Drähte oder Fasern haben vorzugsweise einen Durchmesser im Bereich von 50 - 300 µm und können auch zu Geweben oder Matten verwoben werden. Das so gewonnene Halbzeug wird in einem weiteren Schritt in eine Matrix aus kristallinem Magnesium oder einer kristallinen Magnesium-Legierung eingebettet. Der dadurch entstehende Verbundwerkstoff zeigt im Vergleich zu den beiden Einzelkomponenten ein deutlich verändertes mechanisches Verhalten bei leicht veränderter Korrosionskinetik. Hierbei kann gegebenenfalls die Degradationskinetik noch dadurch beeinflusst werden, dass durch die Auswahl von Faser- und Matrixwerkstoff gezielt eine Potentialdifferenz zwischen Faser und Matrix eingestellt wird.

Ist das Implantat ein vaskuläres Implantat in Form eines Stents, so wird vorzugsweise das Grundgerüst des Stents aus den Fasern oder Drähten der Legierung geformt und dieses Gerüst dann mit einer Matrix aus kristallinem Magnesium oder einer kristallinen Magnesium-Legierung umgossen oder in einem Sinterprozeß eingebettet. Durch den Einsatz vor Fasern und Drähten können insbesondere ballonexpandierbare Stentdesigns verwirklicht werden.

Das Implantat kann weiterhin einen Sinterkörper enthalten, der aus einem Pulver einer der beiden Legierungen gefertigt ist. Durch Zusatz von geeigneten Porenbildnern, die an sich aus dem Stand der Technik bekannt sind, kann eine Porosität des Materials vorgegeben werden. Ein poröses Material bietet sich insbesondere als Wirkstoffdepot an, wobei der in die Poren aufgenommene Wirkstoff während der Biokorrosion der Legierung im Körper freigesetzt wird oder allmählich aus den Poren diffundiert. Denkbar ist auch, eine Oberfläche des medizinischen Implantates im Bereich der Legierung durch Mikrostrukturierung derart zu verändern, dass ein Anhaften von Wirkstoffen oder den Wirkstoff aufnehmendem Beschichtungsmaterial erleichtert wird.

Nach einer weiteren bevorzugten Ausführungsform ist das medizinische Implantat als orthopädisches Implantat, insbesondere zur Osteosynthese ausgebildet. So bieten sich biokorrodierbare orthopädische Implantate insbesondere in der Mund-, Kiefer- und Gesichtschirurgie, der Handchirurgie und als Fixationsimplantate und -nägel für Frakturen an. Der Vorteil im Vergleich zu permanenten Implantaten liegt unter anderem darin, dass im Vergleich zum Permanentimplantat kein erneuter operativer Eingriff zur Entfernung erforderlich ist und die Verbundwerkstoffe mit amorphen bzw. nanokristallinen Verstärkungsfasern im Vergleich zu kristallinen Werkstoffen gleicher Zusammensetzung bessere Materialeigenschaften besitzen. Gegenüber den bekannten biokorrodierbaren metallischen Implantaten sind für einen solchen Verbundwerkstoff eine höhere Festigkeit und eine veränderte, je nach Anwendung verzögerte oder beschleunigte, Korrosionskinetik zu erwarten. Außerdem kann der Legierungsbereich für die Fasern aus amorphen oder nanokristallinen Legierungen gegenüber vergleichbaren kristallinen Legierungen in einem weiteren Bereich variiert werden und so zum Beispiel besser auf Anforderungen der Biokompatibilität eingegangen werden.

Eine weitere bevorzugte Ausführungsform sieht vor, dass das Implantat zur Behandlung vulnerabler Plaque ausgebildet ist. Diese arteriellen Ablagerungen sind, obwohl die Reduktion des Gefäßdurchmessers oft nur gering ist, verantwortlich für einen Großteil der Herzinfarkte, oft mit letalem Ausgang. Ein in seinem Design an bekannte Stents angelehntes Implantat kann nach dieser Indikation in dem Bereich vulnerabler Plaque platziert werden. Es hat sich gezeigt, dass insbesondere Magnesiumlegierungen bei ihrem Abbau im Körper einen positiven physiologischen Effekt auf vulnerable Plaque ausüben und damit eine prophylaktische Behandlung derselben möglich wird. Insbesondere agieren die zu diesem Zwecke geeigneten Implantate auch als Träger von Wirkstoffen, mit denen den entzündlichen Prozessen bei der Bildung vulnerabler Plaque entgegengewirkt werden kann. Die erhöhte mechanische Belastbarkeit eines Verbundwerkstoffes aus kristalliner Matrix und amorphem oder nanokristallinen Fasern führt hierbei zu einem sicheren Einschluss der vulnerablen Plaque an der Gefäßwand über die gesamte Behandlungsdauer.

Eine weitere bevorzugte Einsatzmöglichkeit medizinischer Implantate mit dem erfindungsgemäßen Verbundwerkstoff liegt in der Kinderheilkunde (Pädiatrie). Insbesondere im vaskulären Einsatz ist die Behandlung von Gefäßen mit Dauerimplantaten bedingt durch das Wachstum nicht möglich oder diese Implantate müssen später operativ entfernt werden. Der klinische Vorteil im Vergleich zu aktuellen Behandlungsmethoden mit permanenten Implantaten ist offensichtlich.

Nachfolgend wird die Erfindung anhand von Ausführungsbeispielen und den dazugehörigen Zeichnungen näher erläutert. Es zeigen:
- Fig. 1: eine schematische Darstellung des Prinzips eines Schmelzspinners zur Herstellung amorpher Legierungen;
- Fig. 2a, 2b: eine Draufsicht und einen hieraus vergrößerten Teilausschnitt eines vaskulären Implantats in Form eines ballonexpandierbaren Stents;
- Fig. 3a, 3b: ein vaskuläres Implantat mit einer Fasermatte aus einer amorphen Legierung und
- Fig. 4: ein orthopädisches Implantat zur Osteosynthese von Fragmentfrakturen.

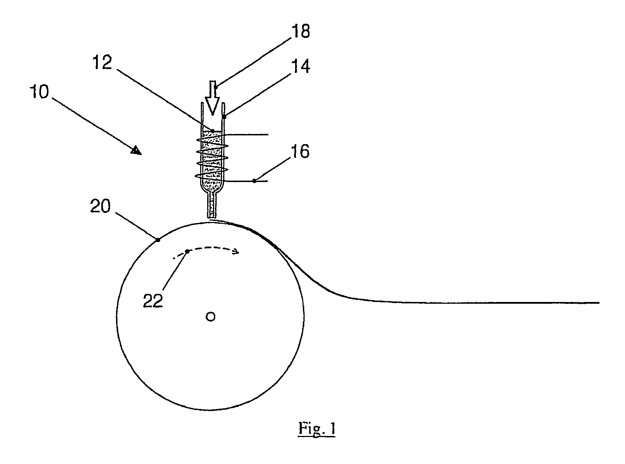

Fig.1 zeigt stark schematisiert die Arbeitsweise eines Schmelzspinners 10 zur Herstellung rasch erstarrter amorpher Legierungen. Eine Schmelze 12 wird in einem Probenbehälter 14, der mit einem Heizer 16 beheizbar ist, bereitgestellt. Durch Druckbeaufschlagung (dargestellt durch den Pfeil 18) tritt die Schmelze 12 aus einer Bohrung am unteren Ende des Probenbehälters 14 aus und fällt auf einen rasch rotierenden, gekühlten Zylinder 20 aus Kupfer (eine Rotationsrichtung des Zylinders 20 ist durch den Pfeil 22 angedeutet). In Folge der sehr raschen Auskühlung des Materials kann ein Übergang in den kristallinen Zustand verhindert werden. Kriterien für die Glasbildung sind insbesondere (i) tiefe Eutektika, da dort eine geringe Stabilität des Kristalls bzw. eine höhere Stabilität der Schmelze zu erwarten ist und (ii) ein deutlicher Größenunterschied der beteiligten Atome der Legierung.

Für die Darstellung amorpher oder nanokristalliner Legierungen besonders geeignete Zusammensetzungen können entweder den einschlägigen Datensammlungen zu binären und ternären Legierungssystemen entnommen werden oder experimentell in standardisierter Weise durch gravimetrische Untersuchungen abkühlender Schmelzen in beheizbaren Zentrifugen bestimmt werden. So ist beispielsweise bekannt, dass die binäre Legierung MgZn ein Eutektikum bei 71,9 Gew.% Magnesium und 28,1 Gew.% Zink besitzt. MgZn-Legierungen dieser Zusammensetzung oder einer Zusammensetzung nahe dem Eutektikum lassen sich besonderes einfach als amorphe oder nanokristalline Legierungen herstellen. Es sei jedoch betont, dass für viele medizintechnische Anwendungen nicht unbedingt das thermodynamisch stabilste amorphe Legierungssystem zu wählen ist, sondern auch das Korrosionsverhalten und natürlich die Biokompatibilität Einflussfaktoren für die Wahl der Legierung sind. Die während der Herstellung der medizinischen Implantate auftretenden mechanischen und thermischen Belastungen sind in der Regel so gering, dass ein Auskristallisieren der Legierungen weitestgehend vermieden werden kann.

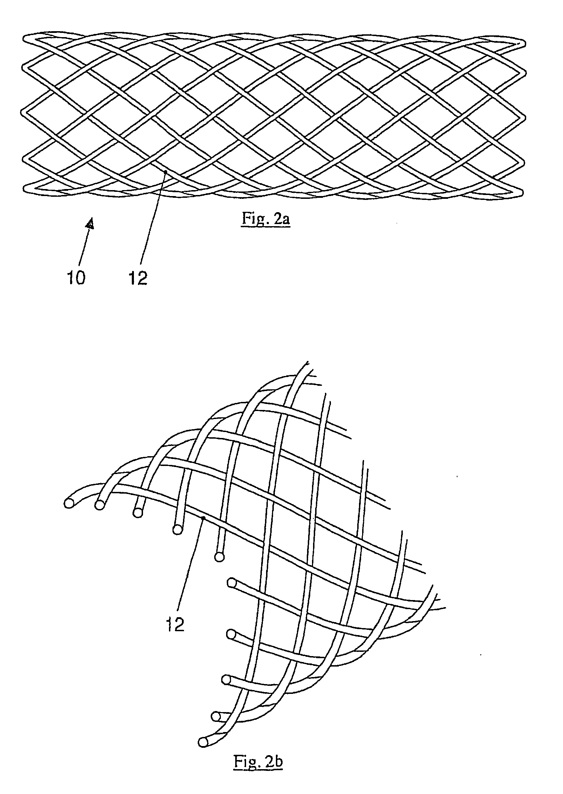

Fig. 2a zeigt schematisch ein vaskuläres Implantat in Form eines ballonexpandierbaren Stents 10 und Fig. 2b einen vergrößerten Teilausschnitt hieraus. Der Stent 10 wird von einem maschenartig verwebten Drahtgeflecht mit spiralförmig umlaufenden Drahtabschnitten 12 gebildet. Das Stentdesign hat vorliegend nur untergeordnete Bedeutung und dient lediglich der Illustration. Das in den Fig. 2a und 2b dargestellte Stentdesign ist ballonexpandierbar ausgelegt, d.h. es kann ausgehend von einem komprimierten ersten Zustand auf mechanischem Weg in einen expandierten zweiten Zustand überführt werden. Derartige Strukturen sind aus dem Stand der Technik hinlänglich bekannt. Zur Realisation muss das eingesetzte Material bestimmten Kriterien wie E-Modul, Brüchigkeit und Festigkeit genügen. Dies kann dadurch erreicht werden, dass die Stege 12 des Stents 10 mit eingebetteten Fasern aus einer biokorrodierbaren amorphen oder nanokristallinen Magnesiumlegierung verstärkt werden. Die Stege 12 haben eine Wandstärke im Bereich von 50 - 300 µm.

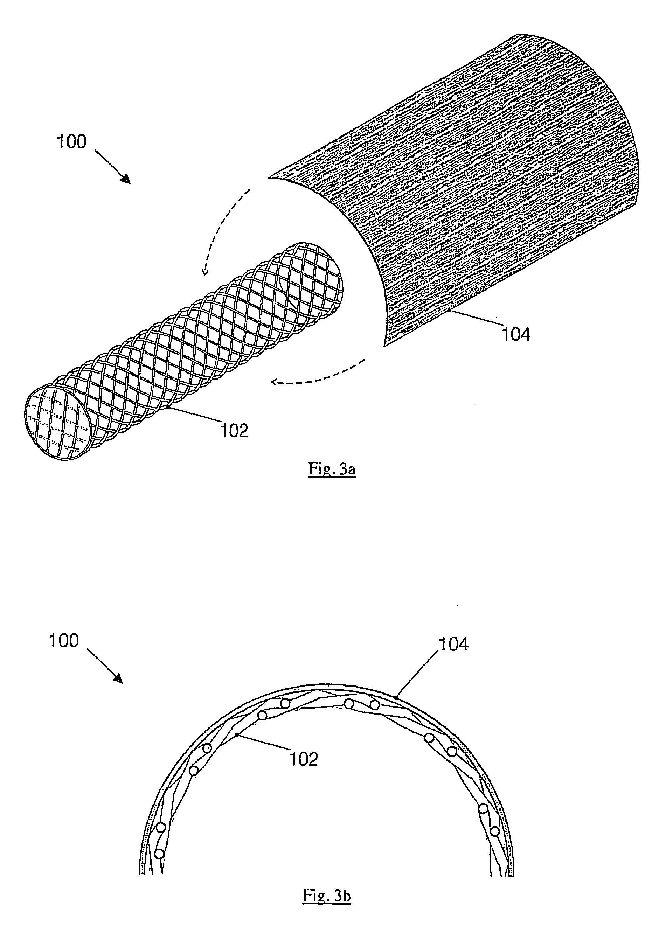

Den Fig. 3a und 3b ist ein weiteres vaskuläres Implantat zu entnehmen, in dem biokorrodierbare amorphe Legierungen Einsatz finden. Das vaskuläre Implantat ist als sogenannter Graftstent 100 ausgebildet und umfasst ein stentartiges Grundgerüst 102 und eine das Grundgerüst 102 aufnehmende dehnbare Hülle 104. Das Grundgerüst 102 kann in der bereits in den Fig. 2a und 2b geschilderten Art und Weise ausgelegt werden, d.h. aus einer biokorrodierbaren amorphen Legierung bestehen. Denkbar ist aber auch ein permanenter Werkstoff, wie medizinischer Stahl. Die Hülle 104 besteht aus dünnen Fasern einer biokorrodierbaren amorphen Magnesiumlegierung, die miteinander verwoben sind. Die Legierungszusammensetzung von Grundgerüst 102 und Hülle 104 kann voneinander abweichen. Die Hülle 104 ist dabei so ausgelegt, dass sie bei Expansion des Grundgerüsts 102 dehnbar ist und eine temporäre Bedeckung oder auch Abdichtung der Gefäßwand, z. B. nach Gefäßruptur, ermöglicht. Der Fig. 3b ist ein Halbquerschnitt durch den Graftstent 100 zu entnehmen, der die relative Lage des Grundgerüstes 102 zur Hülle 104 illustrieren soll.

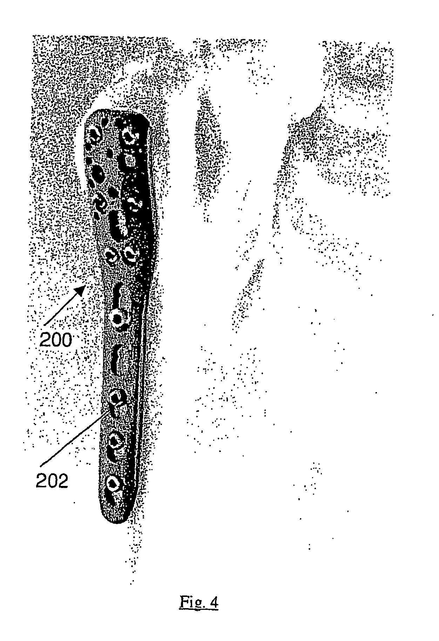

Fig. 4 zeigt ein orthopädisches Implantat zur Osteosynthese von Fragmentfrakturen im Bereich eines Oberarmknochens. Das Implantat 200 besitzt eine der medizinischen Funktion angepasste Geometrie und weist eine Vielzahl von Ausnehmungen auf, durch die Schrauben 202 zur Fixation des Implantats 200 am zu fixierenden Knochen geführt werden. Das Implantat 200 ist aus einem biokorrodierbaren, mit amorphen oder nanokristallinen Fasern verstärkten Verbundwerkstoff aus Magnesium oder einer Magnesiumlegierung geformt, welcher so auszulegen ist, dass das Implantat die mechanischen Belastungen aufnehmen kann und aufgrund der fortschreitenden Biokorrosion frühestens nach etwa 12 Monaten seine mechanische Integrität verliert, da es bis zu diesem Zeitpunkt seine medizinische Funktion erfüllen muss. Auch die Schrauben 202 sind aus einem biokorrodierbaren, mit amorphen oder nanokristallinen Fasern verstärkten Verbundwerkstoff aus Magnesium oder einer Magnesiumlegierung geformt, welche dasselbe Korrosionsverhalten wie das Implantat 200 zeigt, aber nicht zwangsläufig aus derselben Legierungszusammensetzung des Implantats 200 bestehen muss, da andere Anforderungen an die Festigkeit bestehen. Für den Einsatzzweck des Implantats 200 und der Schrauben 202 sind biokorrodierbare, mit amorphen oder nanokristallinen Fasern verstärkte Verbundwerkstoffe hoher Festigkeit bevorzugt.

## Patentansprüche

1. Medizinisches Implantat enthaltend oder bestehend aus einem Verbundwerkstoff, welcher aus einer kristallinen Matrix aus biodegradierbarem Magnesium oder einer biokorrodierbaren Magnesiumlegierung mit amorphen oder nanokristallinen Verstärkungsfasern aus einer magnesiumhaltigen, biokorrodierbaren Legierung, einer anderen biokorrodierbaren Legierung oder einem biologisch nicht abbaubaren Faserwerkstoff besteht, wobei die amorphen oder nanokristallinen Verstärkungsfasern mit einer Hauptkomponente ausgewählt aus der Gruppe Mg, Ca, Fe oder Y hergestellt sind.

2. Implantat nach Anspruch 1 mit amorphen oder nanokristallinen Verstärkungsfasern der Zusammensetzung MgZnX, bei dem (i) ein Anteil von Zn an der Legierung 10 - 40 Gew.% beträgt und (ii) X für ein oder mehrere Elemente ausgewählt aus der Gruppe Ln, Y, Si, Al, Ca steht und ein Anteil von X an der Legierung 0 - 20 Gew.% beträgt.

3. Implantat nach Anspruch 2 mit amorphen oder nanokristallinen Verstärkungsfasern der Zusammensetzung MgZn, bei dem ein Anteil von Zn an der Legierung 26 - 32 Gew.%, insbesondere 28,1 Gew.% beträgt.

4. Implantat nach Anspruch 2 mit einer Legierung der Zusammensetzung MgZnLn, bei dem ein Anteil von Ln an der Legierung 1 - 12 Gew.% beträgt.

5. Implantat nach Anspruch 2 mit amorphen oder nanokristallinen Verstärkungsfasern der Zusammensetzung MgZnY, bei dem ein Anteil von Y an der Legierung 1 - 12 Gew.% beträgt.

6. Implantat nach Anspruch 5, bei dem ein Anteil von Zn an der Legierung 13 - 17 Gew.% und ein Anteil von Ln an der Legierung 8 - 12 Gew.% beträgt oder bei dem ein Anteil von Zn an der Legierung 15 Gew.% und ein Anteil von Ln an der Legierung 10 Gew.% beträgt.

7. Implantat nach Anspruch 2 mit amorphen oder nanokristallinen Verstärkungsfasern der Zusammensetzung MgZnSi, bei der ein Anteil von Si an der Legierung 0,01 - 2 Gew.% beträgt.

8. Implantat nach Anspruch 2 mit amorphen oder nanokristallinen Verstärkungsfasern der Zusammensetzung MgZnAl, bei der ein Anteil von Al an der Legierung 0,01 - 20 Gew.% beträgt.

9. Implantat nach Anspruch 2 mit amorphen oder nanokristallinen Verstärkungsfasern der Zusammensetzung MgZnCa, bei der ein Anteil von Ca an der Legierung 0,01 - 20 Gew.% beträgt.

10. Implantat nach Anspruch 1 mit amorphen oder nanokristallinen Verstärkungsfasern der Zusammensetzung MgY, bei dem ein Anteil von Y an der Legierung 1 - 30 Gew.% beträgt oder bei dem ein Anteil von Y an der Legierung 7 - 11 Gew.%, insbesondere 8,9 Gew.% beträgt.

11. Implantat nach Anspruch 1 mit amorphen oder nanokristallinen Verstärkungsfasern der Zusammensetzung MgYLn, bei der ein Anteil von Y an der Legierung 1 - 30 Gew.% und ein Anteil von Ln an der Legierung 1 - 12 Gew.% beträgt.

12. Implantat nach Anspruch 1 mit amorphen oder nanokristallinen Verstärkungsfasern der Zusammensetzung MgCaAl, bei der ein Anteil von Ca an der Legierung 0,01 - 20 Gew.% und ein Anteil von Al an der Legierung 0,01 - 20 Gew.% beträgt.

13. Implantat nach Anspruch 1 mit amorphen oder nanokristallinen Verstärkungsfasern der Zusammensetzung MgCaSi, bei der ein Anteil von Ca an der Legierung 0,01 - 20 Gew.% und ein Anteil von Si an der Legierung 0,01 - 2 Gew.% beträgt.

14. Implantat nach Anspruch 1 enthaltend Fasern oder Drähte aus der Legierung, bei dem die Fasern oder Drähte als Gewebe oder Matten verwoben sind und einen Durchmesser im Bereich von 50 - 300 µm aufweisen.

15. Implantat nach Anspruch 1 enthaltend Sinterkörper, die aus einem Pulver der Legierung gefertigt sind, wobei der Sinterkörper eine zumindest bereichsweise poröse Oberfläche aufweist.

16. Implantat nach Anspruch 15, bei dem das vaskuläre Implantat ein Stent ist und ein Grundgerüst des Stents aus Fasern oder Drähten der Legierung gemäß Anspruch 14 besteht.

17. Implantat nach Anspruch 16, bei dem der Stent selbstexpandierend ist.

18. Implantat nach einem der Ansprüche 1 bis 15 ausgebildet als orthopädisches Implantat, insbesondere zur Osteosynthese oder ausgebildet zur Fixierung von Gewebe im kardiovaskulären System, oder ausgebildet als Implantat zum Einsatz in der Pädiatrie oder ausgebildet als Implantat zur Behandlung vulnerabler Plaque oder ausgebildet als vaskuläres Implantat.

## Claims

1. A medical implant containing or made of a composite material comprising a crystalline matrix of biocorrodible magnesium or a biocorrodible magnesium alloy having amorphous or nanocrystalline reinforcing fibers made of a magnesium-containing, biocorrodible alloy, another biocorrodible alloy or a biologically non-degradable fibrous material, wherein the amorphous or nanocrystalline reinforcing fibers are produced having a main component selected from the group comprising Mg, Ca, Fe or Y.

2. The implant according to claim 1, comprising amorphous or nanocrystalline reinforcing fibers having the composition MgZnX, in which (i) is a portion of Zn with respect to the alloy of 10 - 40% by weight and (ii) X stands for one or more elements that are selected from the group comprising Ln, Y, Si, Al, Ca, and a portion of X with respect to the alloy is 0 - 20% by weight.

3. The implant according to claim 2, comprising amorphous or nanocrystalline reinforcing fibers having the composition MgZn, in which a portion of Zn with respect to the alloy is 26 - 32% by weight, more particularly 28.1% by weight.

4. The implant according to claim 2, comprising an alloy having the composition MgZnLn, in which a portion of Ln with respect to the alloy is 1 - 12% by weight.

5. The implant according to claim 2, comprising amorphous or nanocrystalline reinforcing fibers having the composition MgZnY, in which a portion of Y with respect to the alloy is 1 - 12% by weight.

6. The implant according to claim 5, in which a portion of Zn with respect to the alloy is 13 - 17% by weight, and a portion of Ln with respect to the alloy is 8 - 12% by weight, or in which a portion of Zn with respect to the alloy is 15% by weight and a portion of Ln with respect to the alloy is 10% by weight.

7. The implant according to claim 2, comprising amorphous or nanocrystalline reinforcing fibers having the composition MgZnSi, in which a portion of Si with respect to the alloy is 0.01 - 2% by weight.

8. The implant according to claim 2, comprising amorphous or nanocrystalline reinforcing fibers having the composition MgZnAl, in which a portion of Al with respect to the alloy is 0.01 - 20% by weight.

9. The implant according to claim 2, comprising amorphous or nanocrystalline reinforcing fibers having the composition MgZnCa, in which a portion of Ca with respect to the alloy is 0.01 - 20% by weight.

10. The implant according to claim 1, comprising amorphous or nanocrystalline reinforcing fibers having the composition MgY, in which a portion of Y with respect to the alloy is 1 - 30% by weight, or in which a portion of Y with respect to the alloy is 7 - 11 % by weight, more particularly 8.9% by weight.

11. The implant according to claim 1, comprising amorphous or nanocrystalline reinforcing fibers having the composition MgYLn, in which a portion of Y with respect to the alloy is 1 - 30% by weight, and a portion of Ln with respect to the alloy is 1 - 12% by weight.

12. The implant according to claim 1, comprising amorphous or nanocrystalline reinforcing fibers having the composition MgCaAl, in which a portion of Ca with respect to the alloy is 0.01 - 20% by weight, and a portion of Al with respect to the alloy is 0.01 - 20% by weight.

13. The implant according to claim 1, comprising amorphous or nanocrystalline reinforcing fibers having the composition MgCaSi, in which a portion of Ca with respect to the alloy is 0.01 - 20% by weight, and a portion of Si with respect to the alloy is 0.01 - 2% by weight.

14. The implant according to claim 1, containing fibers or wires made of the alloy, in which the fibers or wires are woven as fabric or mats and have a diameter in the range of 50 - 300 µm.

15. The implant according to claim 1, which contains a sintered compact made of a powder of the alloy, wherein the sintered compact has a surface that is porous at least in regions.

16. The implant according to claim 15, wherein the vascular implant is a stent and a support frame of the stent comprises fibers or wires of the alloy according to claim 14.

17. The implant according to claim 16, wherein the stent is self-expanding.

18. The implant according to one of the claims 1 to 15 that is designed as an orthopedic implant, more particularly for osteosynthesis, or is designed to immobilize tissue in the cardiovascular system, or is designed as an implant for use in pediatrics, or is designed as an implant for treating vulnerable plaque, or is designed as a vascular implant.

## Revendications

1. Implant médical contenant ou fait d'un matériau composite comprenant une matrice cristalline de magnésium biocorrodable ou d'un alliage de magnésium biocorrodable ayant des fibres de renforcement amorphes ou nanocristallines faites d'un alliage biocorrodable, contenant du magnésium, d'un autre alliage biocorrodable ou d'une matière fibreuse biologiquement non dégradable, les fibres de renforcement amorphes ou nanocristallines étant fabriquées avec un composant principal choisi dans le groupe comprenant Mg, Ca, Fe ou Y.

2. Implant selon la revendication 1, comprenant des fibres de renforcement amorphes ou nanocristallines ayant la composition MgZnX, dans laquelle
(i) une fraction de Zn par rapport à l'alliage représente 10-40 % en poids et
(ii) X représente un ou plusieurs éléments qui sont choisis dans le groupe comprenant Ln, Y, Si, Al, Ca et une fraction de X par rapport à l'alliage représente 0-20 % en poids.

3. Implant selon la revendication 2, comprenant des fibres de renforcement amorphes ou nanocristallines ayant la composition MgZn, dans laquelle une fraction de Zn par rapport à l'alliage représente 23-32 % en poids, plus particulièrement 28,1 % en poids.

4. Implant selon la revendication 2, comprenant un alliage ayant la composition MgZnLn, dans laquelle une fraction de Ln par rapport à l'alliage représente 1-12 % en poids.

5. Implant selon la revendication 2, comprenant des fibres de renforcement amorphes ou nanocristallines ayant la composition MgZnY, dans laquelle une fraction de Y par rapport à l'alliage représente 1-12 % pour cent en poids.

6. Implant selon la revendication 5, dans lequel une fraction de Zn par rapport à l'alliage représente 13-17 % en poids, et une fraction de Ln par rapport à l'alliage représente 8-12 % en poids, ou dans laquelle une fraction de Zn par rapport à l'alliage représente 15 % en poids et une fraction de Ln par rapport à l'alliage représente 10 % en poids.

7. Implant selon la revendication 2, comprenant des fibres de renforcement amorphes ou nanocristallines ayant la composition MgZnSi, dans laquelle une fraction de Si par rapport à l'alliage représente 0,01-2 % en poids.

8. Implant selon la revendication 2, comprenant des fibres de renforcement amorphes ou nanocristallines ayant la composition MgZnAl, dans laquelle une fraction d'Al par rapport à l'alliage représente 0,01-20 % en poids.

9. Implant selon la revendication 2, comprenant des fibres de renforcement amorphes ou nanocristallines ayant la composition MgZnCa, dans laquelle une fraction de Ca par rapport à l'alliage représente 0,01-20 % en poids.

10. Implant selon la revendication 1, comprenant des fibres de renforcement amorphes ou nanocristallines ayant la composition MgY, dans laquelle une fraction de Y par rapport à l'alliage représente 1-30 % en poids, ou dans laquelle une fraction de Y par rapport à l'alliage représente 7-11 % en poids, plus particulièrement 8,9 % en poids.

11. Implant selon la revendication 1, comprenant des fibres de renforcement amorphes ou nanocristallines ayant la composition MgYLn, dans laquelle une fraction de Y par rapport à l'alliage représente 1-30 % en poids, et une fraction de Ln par rapport à l'alliage représente 1-12 % en poids.

12. Implant selon la revendication 1, comprenant des fibres de renforcement amorphes ou nanocristallines ayant la composition MgCaAl, dans laquelle une fraction de Ca par rapport à l'alliage représente 0,01-20 % en poids, et une fraction de Al par rapport à l'alliage représente 0,01-20 %.

13. Implant selon la revendication 1, comprenant des fibres de renforcement amorphes ou nanocristallines ayant la composition MgCaSi, dans laquelle une fraction de Ca par rapport à l'alliage représente 0,01-20 % en poids, et une fraction de Si par rapport à l'alliage représente 0,01-2 % en poids.

14. Implant selon la revendication 1, contenant des fibres ou des fils faits de l'alliage, dans lequel les fibres ou fils sont tissés en tant que tissu ou mats et ont un diamètre se situant dans la plage de 50-300 µm.

15. Implant selon la revendication 1, qui contient un compact fritté fait d'une poudre de l'alliage, le compact fritté ayant une surface qui est poreuse au moins par régions.

16. Implant selon la revendication 15, dans lequel l'implant vasculaire est un stent et un cadre de support du stent comprend des fibres ou fils de l'alliage selon la revendication 14.

17. Implant selon la revendication 16, dans lequel le stent est auto-expansible.

18. Implant selon l'une quelconque des revendications 1 à 15, qui est agencé comme implant orthopédique, plus particulièrement pour l'ostéosynthèse, ou est agencé pour immobiliser du tissu dans le système cardiovasculaire, ou est agencé comme implant pour utilisation en pédiatrie, ou est agencé comme implant pour le traitement de la plaque vulnérable, ou est agencé comme implant vasculaire.
